# EUROPÄISCHE PATENTSCHRIFT

(11) **EP 0 968 177 B1**
(45) Veröffentlichungstag und Bekanntmachung des Hinweises auf die Patenterteilung: **23.01.2002**
(21) Anmeldenummer: 98909420.6
(22) Anmeldetag: 09.02.1998
(51) Int. Cl.: C07C 253/14, C07C 315/04

(54) **VERFAHREN ZUR HERSTELLUNG VON AROMATISCHEN NITRILEN**
METHOD FOR PRODUCING AROMATIC NITRILES
PROCEDE DE FABRICATION DE NITRILES AROMATIQUES

(30) Priorität: 20.02.1997 DE 19706648
(43) Veröffentlichungstag der Anmeldung: 05.01.2000
(73) Patentinhaber: Bayer Aktiengesellschaft, 51368 Leverkusen (DE)
(72) Erfinder: ROCK, Michael-Harold, D-51065 Köln (DE); MARHOLD, Albrecht, D-51373 Leverkusen (DE)
(86) Internationale Anmeldenummer: EP9800696
(87) Internationale Veröffentlichungsnummer: WO9837058

(56) Entgegenhaltungen:
- EP-A- 0 384 392
- EP-A- 0 613 719
- WO-A-96/11906
- CHEMICAL ABSTRACTS, vol. 125, no. 5, 29.Juli 1996 Columbus, Ohio, US; abstract no. 58095, WATABE H. ET AL.: "Preparation of aromatic dicyanates" XP002065875 & JP 08 092 192 A (SUMITOMO CHEMICAL CO.) 9.April 1996
- CHEMICAL ABSTRACTS, vol. 82, no. 13, 31.März 1975 Columbus, Ohio, US; abstract no. 85632, CASSAR L. ET AL.: "Nickel-catalyzed cyanation of aromatic halides" XP002065876 & ADV. CHEM. SER., Nr. 132, 1974, Seiten 252-273, in der Anmeldung erwähnt

## Beschreibung

Die vorliegende Erfindung betrifft ein Verfahren zur Herstellung von aromatischen Nitrilen aus entsprechenden Chloraromaten durch Umsetzung mit Cyaniden. Substituierte Benzonitrile und andere aromatische Nitrile sind bei der Herstellung von pharmazeutischen und landwirtschaftlichen Wirkstoffen häufig eingesetzte Zwischenprodukte.

Es ist bekannt, daß sich Arylhalogenide mit Alkalicyaniden in Gegenwart von Katalysatoren, z.B. Nickelkomplexen, in aromatische Nitrile überführen lassen. In Adv. in Chem. Series **132**: Homogeneous Catalysis II, 252-273 (1974) ist die nickelkatalysierte Cyanierung von Arylhalogeniden mit Alkalicyaniden eingehend beschrieben. Als Katalysatoren wurden Ni(0)- oder Ni(II)-Komplexe mit im allgemeinen ein- oder zweizähnigen Arylphosphinliganden eingesetzt. Als Lösungsmittel wurden Alkohole wie Methanol und Ethanol und dipolar-aprotische Lösungsmittel wie Dimethylformamid eingesetzt. Die Reaktion kann nur mit guten Ausbeuten durchgeführt werden, wenn die Cyanidionenkonzentration genau kontrolliert wird. Nur für die Cyanierung von unsubstituiertem Chlorbenzol ist auch Aceton als Lösungsmittel eingesetzt worden. Dabei wurde bei einem Umsatz von 58% eine Selektivität von 82% erreicht und 10% eines unerwünschten Kupplungsproduktes (Biphenyl) erhalten. Für ein im technischen Maßstab durchzuführendes Verfahren sind das sehr ungünstige Werte. Später ist festgestellt worden, daß der Katalysator bei höheren Cyanidkonzentrationen irreversibel cyaniert wird (s. Bull. Chem. Soc. Jpn. **61**, 1985 (1988) und J. Organomet. Chem. **173**, 335 (1979)).

Für gegebenenfalls durch Halogen, Formyl oder Trifluormethyl substituiertes Chlor- oder Brombenzol ist die Cyanierung in Gegenwart von in situ aus Nickelchlorid hergestellten Katalysatoren in der EP-A 384 392 beschrieben. Die Reaktion wird in wasserfreien niedrigeren Alkoholen oder dipolar aprotischen Lösungsmitteln durchgeführt. Die Ausbeuten sind hierbei häufig nicht zufriedenstellend. Nachteilig ist auch in vielen Fällen eine als Nebenreaktion ablaufende Dehalogenierung. Die bevorzugten Lösungsmittel sind - Acetonitril wegen seiner Giftigkeit und Tetrahydrofuran wegen seiner Neigung zur Peroxidbildung - technisch nur unter großem Aufwand handhabbar.

Für 2-Thio-3-amino-chlorbenzol ist die Cyanierung in Gegenwart von Nickelphosphin-Katalysatoren in aprotisch polaren Lösungsmitteln in der WO 96/11906 beschrieben. Abgesehen. von dem speziellen Substitutionsmuster des Substrats werden mit 11 bis 22 Mol-% Tetrakis-triphenylphosphin-nickel unwirtschaftlich hohe Katalysatormengen eingesetzt. Auch in der EP-A 613 720 ist ein Verfahren zur Herstellung von aromatischen Nitrilen beschrieben, bei dem als Lösungsmittel Tetrahydrofuran mit den o.a. Nachteilen bevorzugt eingesetzt wird.

Es wurde nun ein Verfahren zur Herstellung von aromatischen Nitrilen der Formel

Ar―CN (I),

in der
- Ar: für substituiertes Phenyl oder substituiertes Naphthyl oder gegebenenfalls substituiertes Biphenyl, gegebenenfalls substituiertes Phenyl, das einen ankondensierten 3- bis 5-gliedrigen Heterocyclus enthält, oder gegebenenfalls substituiertes 5 bis 10 Ringglieder enthaltendes Hetaryl steht, wobei als Substituenten bis zu fünf gleiche oder verschiedene aus der Gruppe umfassend C₁-C₁₂-Alkyl, C₁-C₁₂-Halogenalkyl, C₁-C₁₂-Alkoxy, C₁-C₁₂-Halogenalkoxy, C₁-C₆-Alkylsulfonyl, C₁-C₆-Halogenalkylsulfonyl, C₁-C₁₂-Alkylcarbonyl, C₁-C₁₂-Alkoxycarbonyl, Fluor, Formyl, Nitro und Cyano vorliegen können, wobei C₁-C₁₂-Alkyl seinerseits mit 1 bis 4 C₁-C₆-Alkoxygruppen substituiert sein kann,
indem man Chloraromaten der Formel

Ar―Cl (II),

in der
- Ar: die bei Formel (I) angegebene Bedeutung hat,
in Gegenwart von Nickel(0)- und/oder Nickel(II)-Komplexen mit Kalium- und/oder Natriumcyanid umsetzt, das dadurch gekennzeichnet ist, daß man es in Gegenwart eines gegebenenfalls cyclischen Ketons mit mindestens vier Kohlenstoffatomen durchführt.

Erfindungsgemäß einzusetzende Ketone haben gegenüber den bisher verwendeten dipolar aprotischen Lösungsmittel den Vorteil niedriger Toxizität und hoher Stabilität gegenüber Luftsauerstoff. Im Vergleich zu Aceton bei der Cyanierung von unsubstituiertem Chlorbenzol werden beim erfindungsgemäßen Verfahren bei höheren Umsätzen bessere Selektivitäten erzielt und in jedem Fall erheblich weniger unerwünschte Kupplungsprodukte gebildet. Das erfindungsgemäße Verfahren ist deshalb technisch einfacher durchführbar und effektiver als bekannte Verfahren zur Herstellung von aromatischen Nitrilen.

Halogen bei Halogenalkyl, Halogenalkoxy und Halogenalkylsulfonyl steht beispielsweise für Fluor, Chlor oder Brom. Halogenalkyl, Halogenalkoxy und Halogenalkylsulfonyl können mit einem, mit mehreren oder vollständig mit Halogenatomen halogeniert sein.

Bei Phenyl mit ankondensierten 3- bis 5-gliedrigen Heterocyclus kann der ankondensierte Heteroteil z.B. 1 bis 3 Atome aus der Gruppe Stickstoff und Sauerstoff enthalten. Beispiele sind: Benzo-1,3-dioxolyle, insbesondere 2,2-Difluorbenzo-1,3-dioxol-5-yl, Benzo-1,4-dioxinyle, insbesondere 2,2,3,3-Tetrafluorbenzo-1,4-dioxinyl, Benzofuryl, 2,3-Dihydrobenzofuryl, Indolyl und 2,3-Dihydroindolyl.

5 bis 10 Ringglieder enthaltendes Hetaryl kann z.B. im Ring 1 bis 4 Atome aus der Gruppe Stickstoff und Sauerstoff enthalten, wobei maximal 2 Sauerstoffatome vorhanden sind.

Beispiele sind Pyridyl, Pyrimidyl, Purinyl, Furyl und Oxazolyl.

Vorzugsweise steht Ar für substituiertes Phenyl oder substituiertes Naphthyl oder gegebenenfalls substituiertes Biphenyl, gegebenenfalls substituiertes Phenyl, das einen ankondensierten 3- bis 5-gliedrigen Heterocyclus enthält oder gegebenenfalls substituiertes 5 bis 10 Ringglieder enthaltendes Hetaryl, wobei als Substituenten bis zu 4 gleiche oder verschiedene aus der Gruppe umfassend C₁-C₈-Alkyl, C₁-C₈-Fluoralkyl mit bis zu 10 Fluoratomen, C₁-C₈-Chloralkyl mit bis zu 10 Chloratomen, Fluorchloralkyl mit 1 bis 8 C-Atomen und insgesamt bis zu 10 Fluor- und Chloratomen, C₁-C₈-Alkoxy, C₁-C₈-Fluoralkoxy mit bis zu 10 Fluoratomen, C₁-C₈-Chloralkoxy mit bis zu 10 Chloratomen, C₁-C₈-Fluorchloralkoxy mit insgesamt bis zu 10 Fluor- und Chloratomen, C₁-C₄-Alkylsulfonyl, C₁-C₄-Fluoralkylsulfonyl mit bis zu 5 Fluoratomen, C₁-C₄-Chloralkylsulfonyl mit bis zu 5 Chloratomen, Fluorchloralkylsulfonyl mit bis 1 bis 4 C-Atomen und insgesamt bis zu 5 Fluor- und Chloratomen, C₁-C₄-Alkylcarbonyl, C₁-C₄-Alkoxycarbonyl, Fluor, Formyl, Nitro oder Cyano, wobei C₁-C₈-Alkyl seinerseits mit 1 bis 3 C₁-C₄-Alkoxygruppen substituiert sein kann.

Besonders bevorzugt steht Ar für Phenyl, das mit 1 bis 3 Fluoratomen, 1 bis 2 C₁-C₂-Fluoralkylgruppen, 1 bis 2 C₁-C₂-Fluoralkoxygruppen, 1 bis 2 C₁-C₂-Fluoralkylsulfonylgruppen oder 1 bis 2 Formylgruppen und gegebenenfalls zusätzlich mit 1 bis 2 C₁-C₄-Alkylgruppen substituiert ist.

Als erfindungsgemäß einzusetzende, gegebenenfalls cyclische Ketone sind solche mit 4 bis 13 Kohlenstoffatomen bevorzugt. Beispiele sind: Methylethylketon, Methylisobutylketon, Diethylketon, Cyclopentanon, Cyclohexanon und Dicyclohexylketon. Besonders bevorzugt ist Methylethylketon. Zweckmäßigerweise wird das Keton so ausgewählt, daß dessen Siedepunkt sich um mindestens 20°C von dem des entstehenden Nitrils der Formel (I) unterscheidet.

Erfindungsgemäß einzusetzende Ketone können beispielsweise in Mengen von 50 bis 2000 ml, vorzugsweise von 100 bis 500 ml, jeweils bezogen auf 1 Mol Chloraromat eingesetzt werden.

Als Nickel (0)- und Nickel(II)-Komplexe kommen z.B. katalytisch wirksame Verbindungen der Formel (III) infrage

NiL¹L²L³ ₙL⁴ ₘ (III),

in der entweder
i) n und m jeweils unabhängig voneinander für Null oder 1 stehen und
   - L¹, L², L³ und L⁴: unabhängig voneinander für Phosphin-Liganden PQ₃ stehen, worin
   - Q: jeweils unabhängig voneinander für C₁-C₈-Alkyl, C₃-C₈-Cycloalkyl, gegebenenfalls durch C₁-C₈-Alkyl, C₁-C₄-Hydroxyalkyl, C₁-C₆-Alkoxy, Di-C₁-C₄-Alkylamino-C₁-C₄-alkyl, Fluor, Hydroxysulfonyl oder Di-C₁-C₄-alkylamino substituiertes Phenyl steht, oder
   - L¹ und L² und/oder L³ und L⁴: jeweils gemeinsam für zweizähnige Phosphin-Liganden Q₂P-W-PQ₂ stehen, worin
   W für C₁-C₈-Alkylen oder gegebenenfalls durch einen, der für Q = Phenyl oben aufgeführten Substituenten einfach substituiertes Ferrocenyl steht, und
   Q die oben angegebene Bedeutung hat oder in der
ii) n und m jeweils für 1 stehen und
   - L¹ und L²: gemeinsam für einen zweizähnigen Phosphin-Liganden Q¹Q²P-W¹-PQ³Q⁴ stehen, worin
   Q¹, Q², Q³ und Q⁴ unabhängig voneinander für durch Halogen, C₁-C₄-Alkyl, C₁-C₄-Halogenalkyl, Cyano, Amino, C₁-C₄-Alkylamino, Di-C₁-C₄-alkyl-amino, C₁-C₄-Alkoxy oder C₁-C₄-Alkylthio substituiertes Phenyl oder 5 bis 10 Ringglieder und N, O und/oder S-Atome enthaltendes Hetaryl stehen,
   W¹ für ein Metallocen-Biradikal, C₃-C₈-Alkylen oder die Gruppe -Ar¹-(R)ₚ-Ar¹- steht, wobei
   Ar¹ für Phenyl oder 5 bis 10 Ringglieder und N, O und/oder S-Atomc enthaltendes Hetaryl steht,
   p für Null oder 1 steht und
   R für C₁-C₈-Alkylen steht, das gegebenenfalls durch 1 bis 3 Heteroatome aus der Reihe Sauerstoff oder Schwefel unterbrochen ist,
   - L³: für C₁-C₄-Alkyl oder gegebenenfalls ein- bis fünffach unabhängig voneinander durch C₁-C₈-Alkyl, C₁-C₈-Alkoxy, C₁-C₈-Alkylthio, C₁-C₈-Halogenalkyl, C₁-C₈-Halogenalkoxy, Halogen, Cyano, Formyl, C₁-C₈-Alkylcarbonyl, C₁-C₈-Alkoxycarbonyl, substituiertes Phenyl, Phenylcarbonyl, Phenoxy, Phenoxycarbonyl oder Phenyl-C₁-C₄-alkyl steht und
   - L⁴: für Chlor, Brom, Iod, C₁-C₄-Alkyl oder gegebenenfalls durch C₁-C₄-Alkyl substituiertes Aryl oder 5 bis 10 Ringglieder und N-, O- und/oder S-Atome enthaltendes Hetaryl steht, oder
   - L³ und L⁴: gemeinsam für ein Dien R¹-CH-(CH₂)_{q}-CH=CH-R² stehen, worin
   q für 1, 2, 3 oder 4 steht, und
   R¹ und R² jeweils unabhängig voneinander für C₁-C₄-Alkyl oder gemeinsam für C₁-C₄-Alkylen stehen.

Sofern W¹ ein Metallocen-Biradikal darstellt ist Ferrocen bevorzugt.

In den Definitionen für die Formel (III) steht Aryl beispielsweise für Phenyl oder Naphthyl und Heteroaryl beispielsweise für Thienyl, Pyridyl, Furyl, Pyrrolyl, *N*-(C₁-C₄-Alkyl)-pyrrolyl oder Thiazolyl.

Besonders geeignet als Nickelverbindung der Formel (III) ist Tris-triphenylphosphin-nickel.

Die Herstellung von zur Durchführung des erfindungsgemäßen Verfahrens geeigneten Nickelverbindungen ist beispielsweise in WO 96/11906, EP-A 613 720 , EP-A 384 392 und der dort zitierten Literatur beschrieben.

In das erfindungsgemäße Verfahren kann man z.B. 0,95 bis 1,5 mol, vorzugsweise 1,0 bis 1,2 mol Natrium- und/oder Kaliumcyanid und 0,001 bis 0,1 mol, vorzugsweise 0,005 bis 0,1 mol Nickelkomplexe, jeweils pro mol Chloraromat der Formel (II) einsetzen.

Die Reaktion wird beispielsweise bei 40 bis 120°C, bevorzugt bei 50 bis 100°C durchgeführt.

Das erfindungsgemäße Verfahren wird üblicherweise unter Normaldruck durchgeführt. Es ist auch möglich, unter vermindertem oder erhöhtem Druck zu arbeiten.

In einer vorteilhaften Variante des erfindungsgemäßen Verfahrens verwendet man Nickel(0)-Komplexe, die erst unmittelbar vor der Cyanierung in dem erfindungsgemäßen einzusetzenden Lösungsmittel aus einem Nickel(II)-Vorläufer durch Reduktion mit beispielsweise unedlen Metallen wie Magnesium, Zink oder Mangan, gegebenenfalls in Gegenwart eines Überschusses eines Komplexliganden, hergestellt werden.

In einer besonders bevorzugten Variante stellt man zunächst Nickel-bis-triphenylphosphindichlorid aus wasserfreiem Nickeldichlorid und Triphenylphosphin in wasserfreiem Dimethylforamid her. Nach Abdestillieren des Lösungsmittels im Vakuum erhält man so den wasserfreien Nickel(II)-Komplex der zur Reaktion im erfindungsgemäßen Lösungsmittel eingesetzt wird. Durch Zugabe von aktiviertem Zinkpulver und Triphenylphosphin erhält man den aktiven Nickelkatalysator. Dazu wird dann der Chloraromat gegeben, nachgerührt und nach Zusatz des Alkalicyanids erwärmt.

Die Aufarbeitung des nach der Umsetzung vorliegenden Reaktionsgemisches kann z.B. erfolgen, indem man die festen Bestandteile abfiltiert, den Filterkuchen mit einem organischen Lösungsmittel, z.B. dem erfindungsgemäß eingesetzten Keton wäscht, die organischen Phasen vereinigt und destillativ aufarbeitet.

Es ist überraschend, daß die Reaktion bei Verwendung von Ketonen mit mindestens vier Kohlenstoffatomen in guten Ausbeuten verläuft, denn die Cyanierung von Chlorbenzol mit Kaliumcyanid in Aceton gelingt vergleichsweise schlecht (s.o.). Außerdem wird bei der erfindungsgemäßen Verwendung der Ketone bei gleicher Katalysatorkonzentration und Reaktionszeit häufig ein deutlich höherer Umsatz erzielt. Schließlich ist die Bildung von unerwünschten Kupplungsprodukten des Biphenyltyps wesentlich geringer.

### Beispiele

### Beispiel 1

### Herstellung von Nickel-bis-triphenylphosphin-dichlorid (Katalysatorvorstufe)

Eine Mischung von 30,1 g wasserarmen Nickeldichlorid (0,31 Gew.-% Wasser nach Karl-Fischer) und 122,5 g Triphenylphosphin in 200 ml Dimethylformamid (DMF) wurden unter Stickstoffatmosphäre 90 Minuten am Rückfluß gekocht. Danach wurde das Lösungsmittel unter vermindertem Druck abdestilliert. Es wurden 172,2 g NiCl₂(PPh₃)₂ als luftbeständiger, aber feuchtigkeitsempfindlicher Feststoff erhalten, der 19,6 g DMF enthielt. Weiteres Einengen lieferte 156,5 g grünen, nahezu DMF-freien Feststoff, der ebenso luftstabil aber feuchtigkeitsempfindlich war.

Beide Produkte (DMF-haltig und DMF-frei) können erfolgreich als Vorstufe für die in-situ-Herstellung eines Katalysators der Zusammensetzung Ni(PPh₃)₃ eingesetzt werden. Im Falle des DMF-freien Produkts beobachtet man einen um ca. 10 % höheren Umsatz der Chloraromaten der Formel (II).

### Beispiel 2

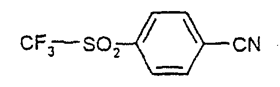

### Herstellung von 4-Trifluormethansulfonylbenzonitril

### a) Erfindungsgemäßes Verfahren in Methylethylketon (MEK)

Eine Mischung von 4 g Nickel-bis-triphenylphosphin-dichlorid, 3 g Triphenylphosphin und 100 ml MEK wurde unter Stickstoffatmosphäre 10 Minuten bei Raumtemperatur gerührt. Danach wurden 1,2 g aktiviertes Zink-Pulver zugesetzt und das Reaktionsgemisch 30 Minuten bei 70°C gerührt. Hiernach wurde auf 25°C abkühlen gelassen und eine Lösung von 92 g 4-Chlor-trifluormethansulfonylbenzol in 50 ml MEK zugetropft. Nach 10 Minuten Rühren wurden 18,5 g Natriumcyanid zugegeben, das Reaktionsgemisch 20 h bei 65 bis 70°C gerührt. Der Umsatz nach dieser Zeit war größer als 98 % (GC). Nach dem Abkühlen auf RT wurde filtriert, der feste Filterrückstand mit 2x mit je 30 ml MEK gewaschen und die vereinigten organischen Phasen unter vermindertem Druck eingeengt. Die Fraktionierung im Vakuum über eine 30 cm-Vigreux-Kolonne und beheizter Destillationsbrücke lieferte 76 g (85 % d.Th.) 4-Trifluormethansulfonylbenzonitril als weißen Feststoff (Siedepunkt bei 0,2 mbar: 115°C). Es wurde keine Bildung von Kupplungsprodukten festgestellt.

### b) Erfindungsgemäßes Verfahren in Methylisobutylketon (MIBK)

Es wurden analog a) verfahren und aus 110 g 4-Chlortrifluormethansulfonylbenzol in 150 ml MIBK 62,1 g (59 % d.Th.) 4-Trifluormethansulfonylbenzonitril erhalten. Der Umsatz nach 20 h betrug ca. 71 % (GC). Es wurde keine Bildung von Kupplungsprodukten festgestellt.

### c) Vergleichsbeispiel

Es wurde analog a) verfahren, jedoch wurde anstelle von MEK das gleiche Volumen DMF eingesetzt. Nach 20 h wurden gaschromatographisch nur 15 % Umsatz zum gewünschten 4-Trifluormethansulfonylbenzonitril gefunden.

### Beispiel 3

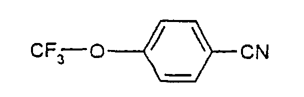

### Herstellung von 4-Trifluormethoxybenzonitril

### a) Erfindungsgemäßes Verfahren in MEK

Eine Mischung von 6,5 g Nickel-bis-triphenylphosphin-dichlorid, 5 g Triphenylphosphin und 120 ml MEK wurde unter Stickstoffatmosphäre 10 Minuten bei Raumtemperatur gerührt. Danach wurden 1,9 g aktiviertes Zink-Pulver zugesetzt und das Reaktionsgemisch 30 Minuten bei 70°C gerührt. Hiernach wurde auf 25°C abkühlen gelassen und 93,25 g 1-Chlor-4-trifluormethoxybenzol zugetropft. Nach 10 Minuten Rühren wurden 24 g Natriumcyanid zugegeben und das Reaktionsgemisch 20 h bei 65 bis 70°C gerührt. Der Umsatz betrug dann ca. 75 % (GC). Nach dem Abkühlen auf Raumtemperatur wurde filtriert, der feste Filterrückstand 2x mit 50 ml MEK gewaschen und die vereinigten organischen Phasen unter vermindertem Druck eingeengt. Die Vakuumdestillation lieferte 58,5 g (66 % d.Th.) 4-Trifluormethoxybenzonitril als farblose Flüssigkeit (Siedepunkt bei 120 mbar: 80°C). Es hatten sich zu 2,6% Kupplungsprodukte gebildet.

### b) Vergleichsbeispiel

In wurde analog a) verfahren, jedoch wurde anstelle von MEK das gleiche Volumen Acetonitril eingesetzt. Nach der gleichen Reaktionszeit wurden nur ca. 35 % Umsatz erreicht (GC). Nach Aufarbeitung wurden 29 g (32,5 % d.Th.) Trifluormethoxybenzonitril erhalten.

### Beispiel 4

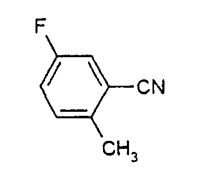

### Herstellung von 5-Fluor-2-methylbenzonitril

### a) Erfindungsgemäßes Verfahren in MEK

Analog zu Beispiel 3 wurden aus 72,5 g 2-Chlor-4-fluortoluol in 100 ml MEK 46,7 g (68 % d.Th) 5-Fluor-2-methylbenzonitril erhalten (Siedepunkt bei 30 mbar: 110°C). Der Umsatz betrug nach 20 h ca. 80 % (GC). Es hatten sich zu 1,2% Kupplungsprodukte gebildet.

### b) Vergleichsbeispiel

Es wurde analog a) verfahren, jedoch wurde anstelle von MEK das gleiche Volumen Tetrahydrofuran eingesetzt. Nach der gleichen Reaktionszeit wurden nur ca. 44 % Umsatz erreicht. Nach Aufarbeitung wurden 26,1 g (38 % d.Th.) 5-Fluor-2-methylbenzonitril erhalten.

### Beispiel 5

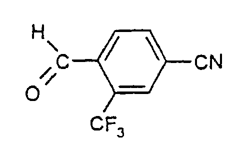

### Herstellung von 4-Cyano-2-trifluormethylbenzaldehyd

Analog zu Beispiel 3 wurden aus 68 g 4-Chlor-2-trifluormethylbenzaldehyd in 100 ml Diethylketon nach Kurzwegdestillation 41,2 g (63,4 % d.Th) 4-Cyano-2-trifluormethylbenzaldehyd als farbloser Feststoff erhalten (Siedepunkt bei 0,5 mbar: 95°C). Der Umsatz war nach 20 h beinnahe vollständig (96 %, GC). Es hatten sich zu 5% Kupplungsprodukte gebildet.

### Beispiel 6

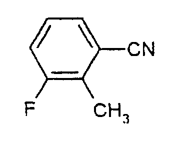

### Herstellung von 3-Fluor-2-methylbenzonitril

Analog zu Beispiel 3 wurden aus 72,25 g 2-Chlor-6-fluortoluol in 100 ml Cyclohexanon 46,2 g (68,5 % d.Th) 3-Fluor-2-methylbenzonitril erhalten (Siedepunkt bei 50 mbar: 104°C). Der Umsatz betrug nach 20 h ca. 90 % (GC). Es hatten sich zu 2% Kupplungsprodukte gebildet.

### Beispiel 7

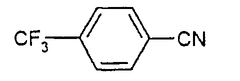

### Herstellung von 4-Trifluormethylbenzonitril

Analog zu Beispiel 3 wurden aus 180,5 g 4-Chlorbenzotrifluorid in 200 ml MEK nach Fraktionierung über eine 30 cm-Vigreux-Kolonne und beheizter Destillationsbrücke 136,6 g (79 % d.Th.) 4-Trifluormethylbenzonitril als farbloser Feststoff erhalten. Es hatten sich zu 2,2% Kupplungsprodukte gebildet.

### Beispiel 8

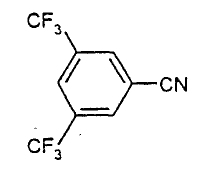

### Herstellung von 3,5-Bis-(trifluormethyl)-benzonitril

Analog zu Beispiel 3 wurden aus 124 g l-Chlor-3,5-bis-(trifluormethyl)-benzol in 120 ml Ethylmethylketon 61,4 g (51,2% d.Th.) 3.5-Bis-(trifluormethyl)-benzonitril erhalten (Kp_{45 mbar}: 88°C). Der Umsatz betrug nach 20 Stunden ca. 60%. Es hatten sich zu 4,5% Kupplungsprodukte gebildet.

### Beispiel 9

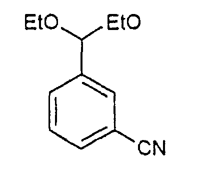

### Herstellung von 3-Diethoxymethyl-benzonitril

Analog zu Beispiel 3a) wurden aus 107,25 g 3-Chlor-diethoxymethyl-benzol in 100 ml MEK 83,3 g (81,2% d.Th.) 3-Diethoxymethyl-benzonitril erhalten (Siedepunkt bei 0,5 bar: 110°C). Der Umsatz betrug nach 4 Stunden 95 %.

## Patentansprüche

1. Verfahren zur Herstellung von aromatischen Nitrilen der Formel
Ar―CN (I),
in der
Ar für substituiertes Phenyl oder substituiertes Naphthyl oder gegebenenfalls substituiertes Biphenyl, gegebenenfalls substituiertes Phenyl, das einen ankondensierten 3- bis 5-gliedrigen Heterocyclus enthält, oder gegebenenfalls substituiertes 5 bis 10 Ringglieder enthaltendes Hetaryl steht, wobei als Substituenten bis zu fünf gleiche oder verschiedene aus der Gruppe umfassend C₁-C₁₂-Alkyl, C₁-C₁₂-Halogenalkyl, C₁-C₁₂-Alkoxy, C₁-C₁₂-Halogenalkoxy, C₁-C₆-Alkylsulfonyl, C₁-C₆-Halogenalkylsulfonyl, C₁-C₁₂-Alkylcarbonyl, C₁-C₁₂-Alkoxycarbonyl, Fluor, Formyl, Nitro und Cyano vorliegen können, wobei C₁-C₁₂-Alkyl seinerseits mit 1 bis 4 C₁-C₆-Alkoxygruppen substituiert sein kann,
indem man Chloraromaten der Formel
Ar―Cl (II),
in der
Ar die bei Formel (I) angegebene Bedeutung hat,
in Gegenwart von Nickel(0)- und/oder Nickel(II)-Komplexen mit Kalium- und/oder Natriumcyanid umsetzt, **dadurch gekennzeichnet, daß** man es in Gegenwart eines gegebenenfalls cyclischen Ketons mit mindestens vier Kohlenstoffatomen durchführt.

2. Verfahren nach Anspruch 1, **dadurch gekennzeichnet, daß** in den Formeln
Ar steht für substituiertes Phenyl oder substituiertes Naphthyl oder gegebenenfalls substituiertes Biphenyl, gegebenenfalls substituiertes Phenyl, das einen ankondensierten 3- bis 5-gliedrigen Heterocyclus enthält oder gegebenenfalls substituiertes 5 bis 10 Ringglieder enthaltendes Hetaryl, wobei als Substituenten bis zu 4 gleiche oder verschiedene aus der Gruppe umfassend C₁-C₈-Alkyl, C₁-C₈-Fluoralkyl mit bis zu 10 Fluoratomen, C₁-C₈-Chloralkyl mit bis zu 10 Chloratomen, Fluorchloralkyl mit 1 bis 8 C-Atomen und insgesamt bis zu 10 Fluor- und Chloratomen, C₁-C₈-Alkoxy, C₁-C₈-Fluoralkoxy mit bis zu 10 Fluoratomen, C₁-C₈-Chloralkoxy mit bis zu 10 Chloratomen, C₁-C₈-Fluorchloralkoxy mit insgesamt bis zu 10 Fluor- und Chloratomen, C₁-C₄-Alkylsulfonyl, C₁-C₄-Fluoralkylsulfonyl mit bis zu 5 Fluoratomen, C₁-C₄-Chloralkylsulfonyl mit bis zu 5 Chloratomen, Fluorchloralkylsulfonyl mit bis 1 bis 4 C-Atomen und insgesamt bis zu 5 Fluor- und Chloratomen, C₁-C₄-Alkylcarbonyl, C₁-C₄-Alkoxycarbonyl, Fluor, Formyl, Nitro oder Cyano, wobei C₁-C₈-Alkyl seinerseits mit 1 bis 3 C₁-C₄-Alkoxygruppen substituiert sein kann.

3. Verfahren nach Ansprüchen 1 und 2, **dadurch gekennzeichnet, daß** in den Formeln Ar für Phenyl, das mit mindestens 1 bis 3 Fluoratomen, 1 bis 2 C₁-C₂-Fluoralkylgruppen, 1 bis 2 C₁-C₂-Fluoralkoxygruppen, 1 bis 2 C₁-C₂-Fluoralkylsulfonylgruppen oder 1 bis 2 Formylgruppen steht und gegebenenfalls zusätzlich mit 1 bis 2 C₁-C₄-Alkylgruppen substituiert ist.

4. Verfahren nach Ansprüchen 1 bis 3, **dadurch gekennzeichnet, daß** die gegebenenfalls cyclischen Kctone 4 bis 13 Kohlenstoffatomen enthalten.

5. Verfahren nach Ansprüchen 1 bis 4, **dadurch gekennzeichnet, daß** das Keton ausgewählt wird aus der Gruppe Methylethylketon, Methylisobutylketon, Diethylketon, Cyclopentanon, Cyclohexanon und Dicyclohexylketon.

6. Verfahren nach Ansprüchen 1 bis 5, **dadurch gekennzeichnet, daß** man als Nickel(0)- und/oder Nickel(II)-Komplexe katalytisch wirksame Verbindungen der Formel (III) verwendet
NiL¹L²L³ ₙL⁴ ₘ (III),
in der entweder
i) n und m jeweils unabhängig voneinander für Null oder 1 stehen und
L¹, L², L³ und L⁴ unabhängig voneinander für Phosphin-Liganden PQ₃ stehen, worin
Q jeweils unabhängig voneinander für C₁-C₈-Alkyl, C₃-C₈-Cycloalkyl, gegebenenfalls durch C₁-C₈-Alkyl, C₁-C₄-Hydroxyalkyl, C₁-C₆-Alkoxy, Di-C₁-C₄-Alkylamino-C₁-C₄-alkyl, Fluor, Hydroxysulfonyl oder Di-C₁-C₄-alkylamino substituiertes Phenyl steht, oder
L¹ und L² und/oder L³ und L⁴ jeweils gemeinsam für zweizähnige Phosphin-Liganden Q₂P-W-PQ₂ stehen, worin
W für C₁-C₈-Alkylen oder gegebenenfalls durch einen, der für Q = Phenyl oben aufgeführten, Substituenten einfach substituiertes Ferrocenyl steht, und
Q die oben angegebene Bedeutung hat oder in der
ii) n und m jeweils für 1 stehen und
L¹ und L² gemeinsam für einen zweizähnige Phosphin-Liganden Q¹Q²P-W¹-PQ³Q⁴ stehen, worin
Q¹, Q², Q³ und Q⁴ unabhängig voneinander für durch Halogen, C₁-C₄-Alkyl, C₁-C₄-Halogenalkyl, Cyano, Amino, C₁-C₄-Alkylamino, Di-C₁-C₄-alkyl-amino, C₁-C₄-Alkoxy oder C₁-C₄-Alkylthio substituiertes Phenyl oder 5 bis 10 Ringglieder und N, O und/oder S-Atome enthaltendes Hetaryl stehen,
W¹ für ein Metallocen-Biradikal, C₃-C₈-Alkylen oder die Gruppe -Ar¹-(R)ₚ-Ar¹- steht, wobei
Ar¹ für Phenyl oder 5 bis 10 Ringglieder und N, O und/oder S-Atome enthaltendes Hetaryl steht,
p für Null oder 1 steht und
R für C₁-C₈-Alkylen steht, das gegebenenfalls durch 1 bis 3 Heteroatome aus der Reihe Sauerstoff oder Schwefel unterbrochen ist,
L³ für C₁-C₄-Alkyl oder gegebenenfalls ein- bis fünffach unabhängig voneinander durch C₁-C₈-Alkyl, C₁-C₈-Alkoxy, C₁-C₈-Alkylthio, C₁-C₈-Halogenalkyl, C₁-C₈-Halogenalkoxy, Halogen, Cyano, Formyl, C₁-C₈-Alkylcarbonyl, C₁-C₈-Alkoxycarbonyl, substituiertes Phenyl, Phenylcarbonyl, Phenoxy, Phenoxycarbonyl oder Phenyl-C₁-C₄-alkyl steht und
L⁴ für Chlor, Brom, lod, C₁-C₄-Alkyl oder gegebenenfalls durch C₁-C₄-Alkyl substituiertes Aryl oder 5 bis 10 Ringglieder und N-, O- und/oder S-Atom enthaltendes Hetaryl steht, oder
L³ und L⁴ gemeinsam für ein Dien R¹-CH-(CH₂)_{q}-CH=CH-R² stehen, worin
q für 1, 2, 3 oder 4 steht, und
R¹ und R² jeweils unabhängig voneinander ein C₁-C₄-Alkyl oder gemeinsam für C₁-C₄-Alkylen stehen.

7. Verfahren nach Ansprüchen 1 bis 6, **dadurch gekennzeichnet, daß** man 0,95 bis 1,5 mol Natrium- und/oder Kaliumcyanid und 0,001 bis 0,1 mol Nickelkomplexe, jeweils pro Mol Chloraromat der Formel (II) einsetzt.

8. Verfahren nach Ansprüchen 1 bis 7, **dadurch gekennzeichnet, daß** man es bei 40 bis 120°C durchführt.

9. Verfahren nach Ansprüchen 1 bis 8, **dadurch gekennzeichnet, daß** man Nickel(0)-Komplexe einsetzt, die erst unmittelbar vor der Cyanierung im einzusetzenden Lösungsmittel aus einem Nickel(II)-Vorläufer durch Reduktion hergestellt werden.

10. Verfahren nach Ansprüchen 1 bis 9, **dadurch gekennzeichnet, daß** man das nach der Umsetzung vorliegende Reaktionsgemisch aufarbeitet, indem man die festen Bestandteile abfiltriert, den Filterkuchen mit einem organischen Lösungsmittel wäscht, die organischen Phasen vereinigt und destillativ aufarbeitet.

## Claims

1. Process for the production of aromatic nitriles having the formula
Ar―CN (I)
in which
Ar stands for substituted phenyl or substituted naphthyl or optionally substituted biphenyl, optionally substituted phenyl containing a fused 3 to 5-member heterocyclus, or optionally substituted hetaryl containing 5 to 10 ring members, whereby there can be up to five identical or different substituents from the group comprising C₁-C₁₂ alkyl, C₁-C₁₂ haloalkyl, C₁-C₁₂ alkoxy, C₁-C₁₂ haloalkoxy, C₁-C₆ alkyl sulfonyl, C₁-C₆ haloalkyl sulfonyl, C₁-C₁₂ alkyl carbonyl, C₁-C₁₂ alkoxy carbonyl, fluorine, formyl, nitro and cyano, whereby C₁-C₁₂ alkyl can in turn be substituted with 1 to 4 C₁-C₆ alkoxy groups,
by reacting chlorine aromatics having the formula
Ar―Cl (II),
in which
Ar has the meaning stated for formula (I)
in the presence of nickel(0) and/or nickel(II) complexes with potassium and/or sodium cyanide, **characterised in that** the reaction is performed in the presence of an optionally cyclic ketone having at least four carbon atoms.

2. Process according to claim 1, **characterised in that** in the formulae
Ar stands for substituted phenyl or substituted naphthyl or optionally substituted biphenyl, optionally substituted phenyl containing a fused 3 to 5-member heterocyclus, or optionally substituted hetaryl containing 5 to 10 ring members, whereby up to four identical or different substituents from the group comprising C₁-C₈ alkyl, C₁-C₈ fluoroalkyl having up to 10 fluorine atoms, C₁-C₈ chloroalkyl having up to 10 chlorine atoms, fluorochloroalkyl having 1 to 8 C atoms and a total of up to 10 fluorine and chlorine atoms, C₁-C₈ alkoxy, C₁-C₈ fluoroalkoxy having up to 10 fluorine atoms, C₁-C₈ chloroalkoxy having up to 10 chlorine atoms, C₁-C₈ fluorochloroalkoxy having a total of up to 10 fluorine and chlorine atoms, C₁-C₄ alkyl sulfonyl, C₁-C₄ fluoroalkyl sulfonyl having up to 5 fluorine atoms, C₁-C₄ chloroalkyl sulfonyl having up to 5 chlorine atoms, fluorochloroalkyl sulfonyl having up to 1 to 4 C atoms and a total of up to 5 fluorine and chlorine atoms, C₁-C₄ alkyl carbonyl, C₁-C₄ alkoxy carbonyl, fluorine, formyl, nitro or cyano, whereby C₁-C₈ alkyl can in turn be substituted with 1 to 3 C₁-C₄ alkoxy groups.

3. Process according to claims 1 and 2, **characterised in that** in the formulae Ar stands for phenyl that is substituted with at least 1 to 3 fluorine atoms, 1 to 2 C₁-C₂ fluoroalkyl groups, 1 to 2 C₁-C₂ fluoroalkoxy groups, 1 to 2 C₁-C₂ fluoroalkyl sulfonyl groups or 1 to 2 formyl groups and optionally additionally with 1 to 2 C₁-C₄ alkyl groups.

4. Process according to claims 1 to 3, **characterised in that** the optionally cyclic ketones contain 4 to 13 carbon atoms.

5. Process according to claims 1 to 4, **characterised in that** the ketone is selected from the group comprising methyl ethyl ketone, methyl isobutyl ketone, diethyl ketone, cyclopentanone, cyclohexanone and dicyclohexyl ketone.

6. Process according to claims 1 to 5, **characterised in that** as nickel(0) and/or nickel(II) complexes catalytically active compounds having the formula (III)
NiL¹L²L³ ₙL⁴ ₘ (III),
are used, in which either
i) n and m mutually independently stand for zero or 1 and
L¹, L², L³ and L⁴ mutually independently stand for phosphine ligands PQ₃, wherein
Q mutually independently stands for C₁-C₈ alkyl, C₃-C₈ cycloalkyl, phenyl optionally substituted by C₁-C₈ alkyl, C₁-C₄ hydroxyalkyl, C₁-C₆ alkoxy, di-C₁-C₄-alkylamino-C₁-C₄-alkyl, fluorine, hydroxysulfonyl or di-C₁-C₄-alkylamino, or
L¹ and L² and/or L³ and L⁴ each jointly stand for bidentate phosphine ligands Q₂P-W-PQ₂, wherein
W stands for C₁-C₈ alkylene or ferrocenyl optionally monosubstituted by a substituent listed above for Q = phenyl, and
Q has the meaning stated above or in which
ii) n and m each stand for 1 and
L¹ and L² jointly stand for a bidentate phosphine ligand Q¹Q²P-W¹-PQ³Q⁴, wherein
Q¹, Q², Q³ and Q⁴ mutually independently stand for phenyl substituted by halogen, C₁-C₄ alkyl, C₁-C₄ haloalkyl, cyano, amino, C₁-C₄ alkylamino, di-C₁-C₄-alkylamino, C₁-C₄ alkoxy or C₁-C₄ alkyl thio or hetaryl containing 5 to 10 ring members and N, O and/or S atoms,
W¹ stands for a metallocene biradical, C₃-C₈ alkylene or the group -Ar¹-(R)ₚ-Ar¹, whereby
Ar¹ stands for phenyl or hetaryl containing 5 to 10 ring members and N, O and/or S atoms,
p stands for zero or 1 and
R stands for C₁-C₈ alkylene which is optionally interrupted by 1 to 3 heteroatoms from the oxygen or sulfur series,
L³ stands for C₁-C₄ alkyl or phenyl, phenyl carbonyl, phenoxy, phenoxycarbonyl or phenyl-C₁-C₄ alkyl, optionally substituted one to five times mutually independently by C₁-C₈ alkyl, C₁-C₈ alkoxy, C₁-C₈ alkyl thio, C₁-C₈ haloalkyl, C₁-C₈ haloalkoxy, halogen, cyano, formyl, C₁-C₈ alkylcarbonyl, C₁-C₈ alkoxycarbonyl, and
L⁴ stands for chlorine, bromine, iodine, C₁-C₄ alkyl or aryl optionally substituted by C₁-C₄ alkyl or hetaryl containing 5 to 10 ring members and N, O and/or S atom, or
L³ and L⁴ jointly stand for an R¹-CH-(CH₂)_{q}-CH=CH-R² diene, wherein
q stands for 1, 2, 3 or 4, and
R¹ and R² stand mutually independently for a C₁-C₄ alkyl or jointly for C₁-C₄ alkylene.

7. Process according to claims 1 to 6, **characterised in that** 0.95 to 1.5 mol sodium and/or potassium cyanide and 0.001 to 0.1 mol nickel complexes are used, in each case per mol of chlorine aromatic having formula (II).

8. Process according to claims 1 to 7, **characterised in that** it is performed at 40 to 120°C.

9. Process according to claims 1 to 8, **characterised in that** nickel(0) complexes are used that are produced only immediately before cyanation in the solvent to be used by reduction from a nickel(II) precursor.

10. Process according to claims 1 to 9, **characterised in that** the reaction mixture obtained after the reaction is recovered by removing the solid components by filtration, washing the filter cake with an organic solvent, combining the organic phases and recovering them by distillation.

## Revendications

1. Procédé pour la préparation de nitriles aromatiques de formule
Ar―CN (I),
dans laquelle
Ar représente un groupe phényle substitué ou naphtyle substitué ou biphényle éventuellement substitué, un groupe phényle éventuellement substitué portant un hétérocycle condensé de 3 à 5 chaînons, ou un groupe hétéroaryle de 5 à 10 chaînons cycliques, éventuellement substitué, les substituants, identiques ou différents et dont le nombre peut aller jusqu'à 5, étant choisis parmi les groupes alkyle en C₁-C₁₂, halogénoalkyle en C₁-C₁₂, alcoxy en C₁-C₁₂, halogénoalcoxy en C₁-C₁₂, alkylsulfonyle en C₁-C₆, halogénoalkylsulfonyle en C₁-C₆, (alkyle en C₁-C₁₂)carbonyle, (alcoxy en C₁-C₁₂)carbonyle, le fluor, les groupes formyle, nitro et cyano, les groupes alkyle en C₁-C₁₂ pouvant eux-mêmes porter 1 à 4 substituants alcoxy en C₁-C₆,
par réaction de dérivés aromatiques chlorés de formule
Ar―Cl (II),
dans laquelle
Ar a les significations indiquées en référence à la formule (I),
en présence de complexes de nickel(0) et ou de nickel(II), avec le cyanure de potassium et/ou de sodium, **caractérisé en ce que** l'on opère en présence d'une cétone éventuellement cyclique à au moins 4 atomes de carbone.

2. Procédé selon la revendication 1, **caractérisé en ce que**, dans les formules,
Ar représente un groupe phényle substitué ou naphtyle substitué ou biphényle éventuellement substitué, un groupe phényle éventuellement substitué portant un hétérocycle condensé de 3 à 5 chaînons ou un groupe hétéroaryle éventuellement substitué de 5 à 10 chaînons cycliques, les substituants, identiques ou différents et dont le nombre peut aller jusqu'à 4, étant choisis parmi les groupes alkyle en C₁-C₈, fluoralkyle en C₁-C₈ avec jusqu'à 10 atomes de fluor, chloralkyle en C₁-C₈ avec jusqu'à 10 atomes de chlore, fluorochloralkyle en C₁-C₈ avec au total jusqu'à 10 atomes de fluor et de chlore, alcoxy en C₁-C₈, fluoralcoxy en C₁-C₈ avec jusqu'à 10 atomes de fluor, chloralcoxy en C₁-C₈ avec jusqu'à 10 atomes de chlore, fluorochloralcoxy en C₁-C₈ avec au total jusqu'à 10 atomes de fluor et de chlore, alkylsulfonyle en C₁-C₄, fluoralkylsulfonyle en C₁-C₄ avec jusqu'à 5 atomes de fluor, chloralkylsulfonyle en C₁-C₄ avec jusqu'à 5 atomes de chlore, fluorochloralkylsulfonyle en C₁-C₄ avec au total jusqu'à 5 atomes de fluor et de chlore, (alkyle en C₁-C₄)carbonyle, (alcoxy en C₁-C₄)carbonyle, le fluor, les groupes formyle, nitro ou cyano, les groupes alkyle en C₁-C₈ pouvant eux-mêmes porter 1 à 3 substituants alcoxy en C₁-C₄.

3. Procédé selon les revendications 1 et 2, **caractérisé en ce que**, dans les formules
Ar représente un groupe phényle substitué par au moins 1 à 3 atomes de fluor, 1 à 2 groupes fluoralkyle en C₁-C₂, 1 à 2 groupes fluoralcoxy en C₁-C₂, 1 à 2 groupes fluoralkylsulfonyle en C₁-C₂ ou 1 à 2 groupes formyle et le cas échéant, en outre, par 1 à 2 groupes alkyle en C₁-C₄.

4. Procédé selon les revendications 1 à 3, **caractérisé en ce que** les cétones éventuellement cycliques contiennent 4 à 13 atomes de carbone.

5. Procédé selon les revendications 1 à 4, **caractérisé en ce que** la cétone est choisie dans le groupe formé par la méthyléthylcétone, la méthylisobutylcétone, la diéthylcétone, la cyclopentanone, la cyclohexanone et la dicyclohexylcétone.

6. Procédé selon les revendications 1 à 5, **caractérisé en ce que** l'on utilise en tant que complexes de nickel(0) et/ou de nickel(II) des composés, possédant une activité catalytique et répondant à la formule (III)
NiL¹L²L³ ₙL⁴ ₘ (III),
dans laquelle
i) n et m sont égaux chacun, indépendamment l'un de l'autre, à 0 ou 1 et
L¹, L², L³ et L⁴ représentent chacun, indépendamment les uns des autres, un ligand du type phosphine PQ₃ pour lequel
les symboles Q représentent chacun, indépendamment les uns des autres, un groupe alkyle en C₁-C₈, cycloalkyle en C₃-C₈, phényle portant lui-même éventuellement des substituants alkyle en C₁-C₈, hydroxyalkyle en C₁-C₄, alcoxy en C₁-C₆, di(alkyle en C₁-C₄)amino-alkyle en C₁-C₄, fluoro, hydroxysulfonyle ou di(alkyle en C₁-C₄)amino, oubien
L¹ et L² et/ou L³ et L⁴ représentent ensemble des ligands bidentiques du type phosphine Q₂P-W-PQ₂ pour lesquels
W représente un groupe alkylène en C₁-C₈ ou un groupe ferrocényle portant éventuellement l'un des substituants mentionnés ci-dessus en référence à Q = phényle, et
Q a les significations indiquées ci-dessus,
ou bien
(ii) n et m sont égaux chacun à 1 et
L¹ et L² forment ensemble un ligand bidentique du type phosphine Q¹Q²P-W¹-PQ³Q⁴ pour lequel
Q¹, Q², Q³ et Q⁴ représentent chacun, indépendamment les uns des autres, un groupe phényle portant des substituants halogéno, alkyle en C₁-C₄, halogénoalkyle en C₁-C₄, cyano, amino, alkylamino en C₁-C₄, di(alkyle en C₁-C₄)amino, alcoxy en C₁-C₄ ou alkylthio en C₁-C₄, ou un groupe hétéroaryle de 5 à 10 chaînons cycliques contenant des atomes de N, O et/ou S,
W¹ représente un biradical de métallocène, un groupe alkylène en C₃-C₈ ou le groupe -Ar¹-(R)ₚ-Ar¹- pour lequel
Ar¹ représente un groupe phényle ou un groupe hétéroaryle de 5 à 10 chaînons cycliques contenant des atomes de N, O et/ou S,
p est égal à 0 ou 1 et
R représente un groupe alkylène en C₁-C₈ éventuellement interrompu par 1 à 3 hétéroatomes choisis parmi l'oxygène et le soufre,
L³ représente un groupe alkyle en C₁-C₄ ou un groupe phényle, phénylcarbonyle, phénoxy, phénoxycarbonyle ou phényl-alkyle en C₁-C₄, portant éventuellement 1 à 5 substituants indépendants les uns des autres choisis parmi les groupes alkyle en C₁-C₈, alcoxy en C₁-C₈, alkylthio en C₁-C₈, halogénoalkyle en C₁-C₈, halogénoalcoxy en C₁-C₈, les halogènes, les groupes cyano, formyle, (alkyle en C₁-C₈)carbonyle, (alcoxy en C₁-C₈)carbonyle, et
L⁴ représente le chlore, le brome, l'iode, un groupe alkyle en C₁-C₄ ou un groupe aryle portant éventuellement des substituants alkyle en C₁-C₄, ou un groupe hétéroaryle de 5 à 10 chaînons cycliques contenant 1 atome de N, O et/ou S, ou bien
L³ et L⁴ représentent ensemble un diène R¹-CH-(CH₂)_{q}-CH=CH-R² pour lequel
q est égal à 1, 2, 3 ou 4 et
R¹ et R² représentent chacun, indépendamment l'un de l'autre, un groupe alkyle en C₁-C₄, ou forment ensemble un groupe alkylène en C₁-C₄.

7. Procédé selon les revendications 1 à 6, **caractérisé en ce que** l'on utilise de 0,95 à 1,5 mol de cyanure de sodium et/ou de potassium et de 0,001 à 0,1 mol de complexes de nickel par mole du dérivé aromatique chloré de formule (II).

8. Procédé selon les revendications 1 à 7, **caractérisé en ce que** l'on opère à des températures de 40 à 120°C.

9. Procédé selon les revendications 1 à 8, **caractérisé en ce que** l'on utilise des complexes du nickel(0) qu'on prépare immédiatement avant la cyanuration dans le solvant à utiliser à partir d'un produit intermédiaire à base de nickel(II) par réduction.

10. Procédé selon les revendications 1 à 9, **caractérisé en ce que**, pour les opérations d'isolement, on filtre les constituants solides du mélange de réaction, on lave le gâteau de filtration avec un solvant organique, on combine les phases organiques et on traite par distillation.
